Europäisches Patentamt

European Patent Office

Office européen des brevets

Veröffentlichungsnummer: **0 286 774**

**A1**

# EUROPÄISCHE PATENTANMELDUNG

Anmeldenummer: 88100095.4

Int. Cl.⁴ **A61K 7/09**

Anmeldetag: 07.01.88

Priorität: 07.03.87 DE 3707415

Veröffentlichungstag der Anmeldung:
**19.10.88 Patentblatt 88/42**

Benannte Vertragsstaaten:
**AT BE CH DE ES FR GB GR IT LI NL SE**

Anmelder: **Wella Aktiengesellschaft**
**Berliner Allee 65**
**D-6100 Darmstadt(DE)**

Erfinder: **Hartmann, Peter**
**Hoffmannstrasse 6**
**D-6100 Darmstadt(DE)**
Erfinder: **Köhler, Joachim, Dr.**
**Waldstrasse 51**
**D-6101 Brensbach/Odw.(DE)**

### Mittel und Verfahren zur dauerhaften Haarverformung.

Gegenstand der Erfindung ist ein 2-Komponenten-Präparat zur dauerhaften Haarverformung auf Thioglykolsäureesterbasis, dessen eine Komponente (A) eine wäßrige Zusammensetzung ist, welche ein Alkali-oder Ammoniumcarbonat oder -hydrogencarbonat enthält, und dessen andere Komponente (B) wasserfrei ist und einen Thioglykolsäureester enthält, welches dadurch gekennzeichnet ist, daß die Komponente (A) 0,1 bis 2,0 Gewichtsprozent Dithiodiglykolsäure oder Dithiodimilchsäure beziehungsweise deren Salz enthält. Die Erfindung betrifft ebenfalls ein Verfahren zur dauerhaften Haarverformung unter Verwendung dieses 2-Komponenten-Präparates.

Das erfindungsgemäße 2-Komponenten-Präparat schäumt beim Vermischen der beiden Komponenten nicht und ermöglicht eine schonende und gleichmäßige Verformung von unterschiedlich stark vorgeschädigtem Haar.

EP 0 286 774 A1

## Mittel und Verfahren zur dauerhaften Haarverformung

Die vorliegende Erfindung betrifft ein Mittel und ein Verfahren zur dauerhaften Haarverformung auf der Basis eines Thioglykolsäureesters und einer Disulfidverbindung.

Für eine schonende dauerhafte Verformung von geschädigtem, insbesondere gebleichtem oder gefärbtem, Haar werden in der Regel schwach sauer bis neutral eingestellte Verformungsmittel verwendet. Hierbei haben sich im Verlauf der letzten 30 Jahre die Thioglykolsäureester als für diesen Zweck am besten geeignete Reduktionsmittel erwiesen.

Jedoch weisen diese Haarverformungsmittel eine Reihe von Nachteilen auf. So verlieren diese Mittel infolge des für die Einstellung des pH-Wertes auf etwa 4,5 bis 7,5 erforderlichen Zusatzes von Puffersubstanzen einen Teil ihrer Verformungswirksamkeit, so daß relativ lange Enwirkungszeiten erforderlich sind. Weiterhin kommt es bei der Verformungsbehandlung von (zum Beispiel durch eine vorherige selektive Bleichung einzelner Haarsträhnen) unterschiedlich stark vorgeschädigtem Haar, infolge einer besseren Durchdringung der geschädigten Haarsträhnen mit dem Verformungsmittel, häufig zu einer uberreduzierung der strapazierten Haarabschnitte, so daß dort keine dauerhafte Verformung erhalten wird, sondern das Haar seine Sprungkraft und Elastizität verliert.

Um eine gleichmäßige Umformung auch bei unterschiedlich stark vorgeschädigtem Haar zu erzielen, wird zum Beispiel in der US-PS 4 273 143 empfohlen, die Verformungsbehandlung nach einem besonderen Verfahren durchzuführen. Herbe wird das Haar zunächst mit einem bestimmte Disulfidderivate (zum Bespiel Salze des Dithiodiglykolsäureglycerinesters) sowie haarpflegende Verbindungen (zum Beispiel quaternäre Ammoniumverbindungen) enthaltenden Vorbehandlungsmittel behandelt. Anschließend wird das Haar mit einem Verformungsmittel, welches neben einem üblichen Reduktionsmittel (zum Beispiel einem Salz oder Ester der Thioglykolsäure) ein Disulfidderivat eines Mercaptans enthält, behandelt. Dieses Disulfidderivat wird vorzugsweise durch Teiloxidation des in dem Verformungsmittel verwendeten Reduktionsmittel hergestellt.

Weiterhin ist es aus den Druckschriften US-PS 2 719 815 und US-PS 3 840 656 bekannt, bei alkalischen Haarverformungsmitteln auf der Basis einer haarkeratinreduzierend wirkenden Mercaptoverbindung durch Zusatz eines der Mercaptoverbindung entsprechenden Disulfids eine Schädigung der Haare bei zu langer Einwirkungszeit des Haarverformungsmittels zu vermeiden.

Die vorstehend genannten Verfahren ermöglichen zwar eine schonendere Verformungsbehandlung, jedoch bewirkt der Zusatz der (dem eingesetzten Reduktionsmittel entsprechenden) Disulfidverbindungen eine deutliche Abschwächung der Verformungswirksamkeit des verwendeten Mittels, wodurch die für eine zufriedenstellende Haarverformung erforderlichen Einwirkungszeiten erheblich verlangert werden.

Werden diese Disulfidverbindungen in höheren Konzentrationen dem Verformungsmittel zugesetzt, so besteht außerdem de Gefahr, daß ein größerer Anteil an gemischten Disulfiden entsteht, die bei der anschließenden oxidativen Nachbehandlung nicht mehr vollständig in Cystindisulfid (Cystein) umgewandelt werden können. Da auf diese Weise nur en Teil der bei der Verformungsbehandlung gespaltenen Disulfdbrückenbindungen des Haarkeratins wieder miteinander verknüpft wird, verschlechtert sich die Haarqualität erheblich.

Ein weiteres Problem bei Haarverformungsmitteln auf Thioglykolsäureesterbasis ist die pH-Wert-Einstellung und Pufferung dieser Mittel. Da handelsübliche Thioglykolsäureester etwa 1 Gewichtsprozent freie Thioglykolsäure enthalten, beträgt der pH-Wert einer wäßrigen Lösung dieser Thioglykolsäureester etwa 2 bis 3,5. Um das gebrauchsfertige Präparat auf den gewünschten pH-Wert von 4,5 bis 7,5 einzustellen, muß der wäßrigen Lösung deshalb ein Alkalisierungsmittel beziehungsweise eine Puffersubstanz zugesetzt werden. Jedoch wird die Verformungswirksamkeit von Verformungsmitteln auf Thioglykolsäureesterbasis durch den Zusatz von Alkalisierungsmitteln beziehungsweise Puffersubstanzen, wie zum Beispiel Aminosäuren oder Natrium-und Dinatriumhydrogenphosphat, erheblich vermindert. Die besten Umformungsergebnisse werden bei Verwendung von Alkali-oder Ammoniumcarbonaten oder - hydrogencarbonaten erzielt.

Da die Thioglykolsäureester nur in wasserfreier Form über einen längeren Zeitraum stabil sind, in wäßrigem Medium hingegen bereits nach kurzer Zeit (etwa 8 bis 12 Stunden), hydrolysieren, sind wäßrige Einkomponentenpräparate, wie sie beispielsweise in der US-PS 4 273 143 beschrieben werden, wegen ihrer stark begrenzten Lagerfähigkeit für eine industrielle Herstellung ungeeignet.

Zur Herstellung von über einen längeren Zeitraum lagerfähigen Präparaten wird bei den heute üblichen Thioglykolsäureesterpräparaten daher die Esterkomponente in wasserfreier Form abgepackt und das gebrauchsfertige Verformungsmittel vor dem Gebrauch durch Vermischen des Thioglykolsäureesters mit der die übrigen Bestandteile enthaltenden wäßrigen Komponente hergestellt.

Ein Nachteil dieser 2-Komponenten-Präparate ist es je doch, daß bei Verwendung von Alkali-oder Ammoniumcarbonaten oder -hydrogencarbonaten als Puffersubstanz beim Zumischen der sauren Thioglykolsäureesterkomponente das Mittel, infolge einer starken $CO_2$-Entwicklung, heftig aufschäumt und hierdurch ein Teil des Verformungsmittels unkontrolliert aus der üblicherweise für das Vermischen der beiden Komponenten verwendeten Auftrageflasche auf den Boden oder die Kleidung des Anwenders spritzt.

Der Erfindung liegt daher die Aufgabe zugrunde, ein lagerfähiges Haarverformungsmittel zur Verfügung zu stellen, das bei normaler Einwirkungsdauer ohne Verwendung eines Vorbehandlungsmittels eine  -schonende und gleichmäßige Umformung von unterschiedlich stark geschädigtem Haar ermöglicht. Eine weitere Aufgabe bestand darin, bei mit Alkali-oder Ammoniumcarbonaten oder -hydrogencarbonaten gepufferten 2-Komponenten-Präparaten auf Thioglykolsäureesterbasis ein Aufschäumen während des Vermischens der beiden Komponenten zu vermeiden.

Es wurde nun gefunden, daß mit Alkali-oder Ammoniumcarbonaten oder -hydrogencarbonaten gepufferte, thioglykolsäureesterhaltige Mittel, die erst unmittelbar vor ihrem Gebrauch durch Vermischen der thioglykolsäureesterhaltigen Komponente mit einer zweiten, bestimmte Disulfidverbindungen enthaltenden, wäßrigen Komponente hergestellt werden, die vorstehend genannte Aufgabe lösen. Diese Mittel schäumen beim Vermischen der beiden Komponenten nicht und ermöglichen eine schonende und gleichmäßige Verformung von unterschiedlich stark vorgeschädigtem Haar, ohne daß eine Verlängerung der Einwirkungszeit oder die Verwendung eines Vorbehandlungsmittels erforderlich ist.

Gegenstand der vorliegenden Erfindung ist daher ein Mittel zur dauerhaften Verformung von Haaren auf der Basis von Thioglykolsäureester in Form eines 2-Komponen ten-Präparates, dessen eine Komponente (A) eine wäßrige Zusammensetzung ist, welche ein Alkali-oder Ammoniumcarbonat oder ein Alkali-oder Ammoniumhydrogencarbonat enthält, und dessen andere Komponente (B) wasserfrei ist und den Thioglykolsäureester enthält, welches dadurch gekennzeichnet ist, daß die Komponente (A) 0.1 bis 2,0 Gewichtsprozent Dithiodiglykolsäure, Dithiodimilchsäure oder deren Salz enthält.

Das erfindungsgemäße Mittel wird unmittelbar vor Gebrauch durch Vermischen der wäßrigen, das Alkali-oder Ammoniumcarbonat oder -hydrogencarbonat sowie bestimmte Disulfidverbindungen enthaltenden, Komponente (A) mit der, den Thioglykolsäureester enthaltenden, wasserfreien Komponente (B) hergestellt.

Die Alkali-oder Ammoniumcarbonate oder -hydrogencarbonate, von denen Ammoniumhydrogencarbonat bevorzugt ist, werden insbesondere in einer Menge von 0,1 bis 1,0 Gewichtsprozent (bezogen auf das gebrauchsfertige Verformungsmittel) verwendet.

Während der Thioglykolsäureester, welcher vorzugsweise der Thioglykolsäuremonoglycerinester ist, in dem gebrauchsfertigen Verformungsmittel zweckmäßigerweise in einer Menge von etwa 5 bis 30 Gewichtsprozent (entsprechend einem Gehalt an freier Thioglykolsäure von 2 bis 16 Gewichtsprozent) enthalten ist, beträgt der Gehalt an der Disulfidverbindung etwa 0,1 bis 2,0 Gewichtsprozent (bezogen auf das gebrauchsfertige Verformungsmittel), wobei der Thioglykolsäureester und die Disulfidverbindung in dem gebrauchsfertigen Verformungsmittel vorzugsweise in einem Gewichtsverhältnis zwischen 15 : 1 und 200 : 1 enthalten sind.

Als Disulfidverbindung werden in dem erfindungsgemäßen Mittel Dithiodiglykolsäure, Dithiodimilchsäure oder deren Salze, insbesondere Dinatriumdithiodiglykolat, Diammoniumdithiodiglykolat, Dinatriumdithiodilactat und Diammoniumdithiodilactat, sowie Mischungen dieser Verbindungen eingesetzt.

Der pH-Wert des gebrauchsfertigen Verformungsmittels beträgt 4,5 bis 7,5.

Das Verformungsmittel kann sowohl in Form einer wäßrigen Lösung oder einer Emulsion als auch in verdickter Form auf wäßriger Basis, insbesondere als Gel, Creme oder Paste, vorliegen.

Selbstverständlich kann das Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Alkalisierungsmittel, wie beispielsweise Ammoniak, Parfumöle, Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netz mittel und Emulgatoren in Konzentrationen von etwa 0,2 bis 30 Gewichtsprozent, während die Verdickungsmittel in einer Menge von etwa 0,5 bis 20 Gewichtsprozent in diesem Mittel enthalten sein können.

3

Weiterhin können diesem Mittel zur Wirkungssteigerung sogenannte Quell-und Penetrationsstoffe, wie zum Beispiel Dipropylenglykolmonomethylether, 2-Pyrrolidon oder Imidazolidin-2-on, in einer Menge von etwa 2 bis 30 Gewichtsprozent zugesetzt werden.

Durch den Zusatz von Dithiodiglykolsäure oder Dithiodimilchsäure oder deren Salzen wird beim Vermischen der beiden Komponenten von mit Alkali-oder Ammoniumcarbonaten oder -hydrogencarbonaten gepufferten 2-Komponenten-Haarverformungsmitteln ein Aufschäumen verhindert und eine sichere Handhabung dieser Präparate gewährleistet.

Das erfindungsgemäße Mittel ermöglicht eine schonende und gleichmäßige Umformung von unterschiedlich stark vorgeschädigtem Haar, ohne die vorgeschädigten Haarpartien zu überkrausen. Auf die bisher übliche Vorbehandlung der Haare mit einem speziellen Vorbehandlungsmittel kann bei Verwendung des erfindungsgemäßen Mittels verzichtet werden, ohne daß hierdurch das Verformungsergebnis verschlechtert wird.

Weiterhin wird durch die Verwendung des erfindungsgemäßen Mittels die Sprungkraft der Locken sowie die Elastizität der Haare verbessert.

Überraschenderweise wird durch den Zusatz von Dithiodiglykolsäure, Dithiodimilchsäure oder deren Salzen die Verformungsgeschwindigkeit nicht herabgesetzt; vielmehr besitzt das erfindungsgemäße Mittel, insbesondere bei vorgeschädigtem Haar, eine gegenüber üblichen Verformungsmitteln auf Thioglykolsäureesterbasis erhöhte Umformungsgeschwindigkeit.

Die vorliegende Erfindung betrifft weiterhin ein Verfahren zur dauerhaften Verformung von Haaren, bei dem man das Haar bevor und/oder nachdem man es in die gewünschte Form bringt mit einem Verformungsmittel behandelt, gegebenenfalls mit Wasser spült, sodann oxidativ nachbehandelt, mit Wasser spült, gegebenenfalls zur Wasserwelle legt und sodann trocknet, welches dadurch gekennzeichnet ist, daß für die Verformungsbehandlung das vorstehend beschriebene, unmittelbar vor dem Gebrauch durch Vermischen der Komponenten (A) und (B) hergestellte, Verformungsmittel verwendet wird.

Bei einer Ausführungsform des erfindungsgemäßen Verfahrens wird das Haar zunächst mit einem Shampoo gewaschen und danach mit Wasser gespült Anschließend wird das handtuchtrockene Haar in einzelne Strähnen aufgeteilt und auf Wickler mit einem Durchmesser von etwa 5 bis 15 Millimetern gewickelt Sodann wird das Haar mit einer für die Haarverformung ausreichenden Menge, vorzugsweise etwa 80 bis 120 Gramm, des beschriebenen erfindungsgemäßen Verformungsmittels behandelt.

In einer weiteren Ausführungsform des erfindungsgemäßen Verfahrens wird das Haar im Anschluß an die Haarwäsche mit einem Teil des Verformungsmittels, vorzugsweise etwa 40 bis 60 Gramm, vorgefeuchtet, in einzelne Strähnen aufgeteilt und auf Wickler gewickelt. Sodann wird das Haar mit dem restlichen Verformunsgmittel, vorzugsweise etwa 40 bis 60 Gramm, nochmals behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur etwa 10 bis 45 Minuten (10 bis 30 Minuten mit Wärmeeinwirkung; 20 bis 45 Minuten ohne Wärmeeinwirkung) beträgt, wird das Haar mit Wasser gespült und dann oxidativ nachbehandelt ("fixiert"). Das Nachbehandlungsmittel wird, je nach Haarfülle, in einer Menge von etwa 80 bis 100 Gramm verwendet.

Für die oxidative Nachbehandlung kann jedes beliebige, bisher für eine derartige Behandlung verwendete, Nachbehandlungsmittel verwendet werden. Beispiele für in einem derartigen Nachbehandlungsmittel verwendbare Oxidationsmittel sind Kalium-und Natriumbromat, Natriumperborat, Harnstoffperoxid und Hydrogenperoxid. Die Konzentration des Oxidationsmittels ist in Abhängigkeit von der Anwendungszeit (in der Regel etwa 5 bis 15 Minuten) und der Anwendungstemperatur unterschiedlich. Normalerweise liegt das Oxidationsmittel in dem wäßrigen Nachbehandlungsmittel in einer Konzentration von etwa 0,5 bis 10 Gewichtsprozent vor. Das Mittel für die oxidative Nachbehandlung kann selbstverständlich weitere Stoffe, wie zum Beispiel Netzmittel, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die nachfolgenden Beispiele sollen den Gegenstand der Erfindung erläutern, ohne ihn hierauf zu beschränken.

**Beispiele**

**Beispiel 1**

Komponente (A):

0,2 g Diammoniumdithiodiglykolat

1,0 g Ammoniumhydrogencarbonat

0,8 g Ammoniak (25-prozentige wäßrige Lösung)

0,3 g Parfümöl

97,7 g Wasser, vollentsalzt

_____

100,0 g

Komponente (B):

30,0 g Thioglykolsäuremonoglycerinester

20,0 g Glycerin

_____

50,0 g

Vor dem Gebrauch werden die Komponente (A) und die Komponente (B) miteinander vermischt, wobei im Gegensatz zu den bisher üblichen 2-Komponenten-Präparaten auf Thioglykolsäureglycerinesterbasis kein starkes Aufschäumen zu beobachten ist. Der pH-Wert dieses Haarverformungsmittels beträgt 6,8.

Unterschiedlich stark vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 20 Minuten lang unter einer Trockenhaube auf eine Temperatur von 40 Grad Celsius erwärmt.

Anschließend wird die Abdeckung entfernt, das Haar mit Wasser gespült und mit 100 g einer 3-prozentigen wäßrigen Hydrogenperoxidlösung oxidativ nachbehandelt. Nach Entfernung der Wickler werden die Haare erneut mit Wasser gespült zur Wasserwelle gelegt und sodann getrocknet.

Als Ergebnis dieser Behandlung wird eine gleichmäßige, natürlich wirkende Umformung der Haare erhalten.

**Beispiel 2**

Komponente (A):

1,0 g Dinatriumdithiodilactat

0,5 g Ammoniak (25-prozentige wäßrige Lösung)

0,3 g Parfümöl

0,2 g Ammoniumhydrogencarbonat

98,0 g Wasser, vollentsalzt .

─────────

100,0 g

Komponente (B):

.

30,0 g Thioglykolsäuremonoglycerinester

20,0 g Glycerin

─────────

50,0 g

Das gebrauchsfertige Haarverformungsmittel wird durch Vermischen der Komponente (A) mit der Komponente (B) hergestellt. Der pH-Wert dieses Haarverformungsmittels beträgt 6.5.

Ein Haar, welches starke Strukturunterschiede aufweist, wird mit einem Shampoo gewaschen und mit Wasser gründlich gespült. Anschließend wird etwa die Hälfte des vorstehenden gebrauchsfertigen Haarverformungsmittels gleichmäßig auf dem frottierten Haar verteilt, das Haar auf Wickler mit einem Durchmesser von 6 Millimetern gewickelt und sodann mit dem restlichen Haarverformungsmittel nochmals befeuchtet. Nach einer Einwirkungszeit von 30 Minuten bei Raumtemperatur wird das Haar mit Wasser gespült und mit 100 g einer 2-prozentigen wäßrigen Hydrogenperoxidlösung oxidativ nachbehandelt. Nach Entfernung der Wickler werden die Haare erneut mit Wasser gespült und danach getrocknet.

Das so behandelte Haar besitzt eine gleichmäßige Krause, deren Locken eine gute Elastizität und Sprungkraft aufweisen.

**Beispiel 3**

Komponente (A):

1,0 g Dinatriumdithiodiglykolat

1,0 g Diammoniumdithiodilactat

2,0 g Ammoniak (25-prozentige wäßrige Lösung)

0,5 g Ammoniumhydrogencarbonat

0,3 g Parfümöl

95,2 g Wasser, vollentsalzt

_____

100,0 g

Komponente (B):

30,0 g Thioglykolsäuremonoglycerinester

20,0 g Glycerin

_____

50,0 g

Kurz vor dem Gebrauch wird die Komponente (A) mit der Komponente (B) zu einem Haarverformungsmittel mit einem pH-Wert von 7,4 vermischt.

Ein Haar von unterschiedlicher Haarqualität wird gewaschen, mit einem Handtuch frottiert und auf Wickler mit einem Durchmesser von 8 Millimetern gewickelt. Anschließend wird das Haar mit dem vorstehend beschriebenen gebrauchsfertigen Haarverformungsmittels gleichmäßig durchfeuchtet. Nach einer Einwirkungszeit von 30 Minuten bei Raumtemperatur wird das Haar mit Wasser gespült und sodann mit 80 g einer 3-prozentigen wäßrigen Hydrogenperoxidlösung oxidativ nachbehandelt. Nach dem Entfernen der Wickler werden die Haare erneut mit Wasser gespült, zur Wasserwelle gelegt und anschließend getrocknet.

Als Ergebnis dieser Behandlung wird ein gleichmäßig verformtes Haar mit hoher Sprungkraft und Elastizität erhalten.

Als Thioglykolsäuremonoglycerinester wurde ein handelsüblicher Thioglykolsäuremonoglycerinester verwendet. Der pH-Wert einer 20-prozentigen wäßrigen Lösung dieses Esters beträgt etwa 2 bis 3,5.

Alle in der vorliegenden Anmeldung enthaltenen Prozentangaben stellen Gewichtsprozente dar.

**Ansprüche**

1. Mittel zur dauerhaften Verformung von Haaren auf der Basis von Thioglykolsäureester in Form eines 2-Komponenten-Präparates, dessen eine Komponente (A) eine wäßrige Zusammensetzung ist, welche ein Alkali-oder Ammoniumcarbonat oder ein Alkali-oder Ammoniumhydrogencarbonat enthält, und dessen andere Komponente (B) wasserfrei ist und den Thioglykolsäureester enthält, dadurch gekennzeichnet, daß die Komponente (A) 0,1 bis 2,0 Gewichtsprozent Dithiodiglykolsäure, Dithiodimilchsäure oder deren Salz enthält.

2. Mittel nach Anspruch 1, dadurch gekennzeichnet, daß das Salz der Dithiodiglykolsäure oder Dithiodimilchsäure das Dinatriumdithiodiglykolat, Diammoniumdithiodiglykolat, Dinatriumdithiodilactat oder Diammonumdithiodilactat ist.

3. Mittel nach Anspruch 1 oder 2, dadurch gekennzeichnet, daß der Thioglykolsäureester in einer Menge von 5 bis 30 Gewichtsprozent, bezogen auf das gebrauchsfertige Verformungsmittel, enthalten ist.

4. Mittel nach einem der Ansprüche 1 bis 3, dadurch gekennzeichnet, daß der Thioglykolsäureester der Thioglykolsäuremonoglycerinester ist.

5. Mittel nach einem der Ansprüche 1 bis 4, dadurch gekennzeichnet, daß die Komponente (A) Ammoniumhydrogencarbonat enthält.

6. Mittel nach einem der Ansprüche 1 bis 4, dadurch gekennzeichnet, daß der pH-Wert des gebrauchsfertigen Verformungsmittels 4,5 bis 7,5 beträgt.

7. Mittel nach einem der Ansprüche 1 bis 6, dadurch gekennzeichnet, daß es den Thioglykolsäureester und die Disulfidverbindung in einem Gewichtsverhältnis von 15 : 1 bis 200 : 1, bezogen auf das gebrauchsfertige Verformungsmittel, enthält.

8. Verfahren zur dauerhaften Verformung von Haaren, bei dem man das Haar bevor und/oder nachdem man es in die gewünschte Form bringt mit einem Verformungsmittel behandelt, mit Wasser spült, sodann oxidativ nachbehandelt, mit Wasser spült, gegebenenfalls zur Wasserwelle legt und sodann trocknet, dadurch gekennzeichnet, daß man als Haarverformungsmittel ein unmittelbar vor dem Gebrauch durch Vermischen der Komponenten (A) und (B) nach einem der Ansprüche 1 bis 7 hergestelltes Mittel verwendet.

9. Verfahren nach Anspruch 8, dadurch gekennzeichnet, daß man das Verformungsmittel 10 bis 45 Minuten lang einwirken läßt.

10. Verfahren nach Anspruch 8 oder 9, dadurch gekennzeichnet, daß man das Verformungsmittel in einer Menge von 80 bis 120 Gramm anwendet.

8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | US-A-4 273 143 (KLEMM et al.) <br> * Ansprüche * <br> --- | 1-10 | A 61 K 7/09 |
| D,A | US-A-2 719 815 (SANDERS) <br> * Ansprüche; Beispiel 1 * <br> --- | 1-10 | |
| D,A | US-A-3 840 656 (KALOPISSIS et al.) <br> * Ansprüche; Beispiel 1 * <br> ----- | 1-10 | |
| | | | **RECHERCHIERTE SACHGEBIETE (Int. Cl.4)** <br><br> A 61 K 7/00 |

Der vorliegende Recherchenbericht wurde für alle Patentansprüche erstellt

| Recherchenort | Abschlußdatum der Recherche | Prüfer |
|---|---|---|
| DEN HAAG | 05-08-1988 | WILLEKENS G.E.J. |